# EUROPEAN PATENT APPLICATION

(11) **EP 2 902 383 A1**
(43) Date of publication of application: **05.08.2015**
(21) Application number: 13842304.1
(22) Date of filing: 22.07.2013
(51) Int. Cl.: C07C 253/30, C07C 255/03, C07C 255/50, C07D 213/84, C07F 1/08

(54) **METHOD FOR PRODUCING COMPLEX COMPOSED OF ORGANIC NITROGEN COMPOUND AND COPPER (I) SALT OF FLUOROOXO ACID**

(30) Priority: 28.09.2012 JP 2012217769
(71) Applicant: Sumitomo Seika Chemicals Co., Ltd., Kako-gun, Hyogo 675-0145 (JP)
(72) Inventor: SAKAUE, Shigeki, Kako-gun Hyogo 675-0145 (JP); FUJIWARA, Takeshi, Kako-gun Hyogo 675-0145 (JP); MATSUO, Nami, Kako-gun Hyogo 675-0145 (JP)
(74) Representative: Webster, Jeremy Mark
(86) International application number: PCT/JP2013/069762
(87) International publication number: WO 2014/050272

(57) **Abstract**

The present invention mainly addresses the problem of providing a method for producing a complex composed of an organic nitrogen compound and a copper (I) salt of a fluorooxo acid with high yield while reducing the corrosion of a reaction vessel. The complex can be produced with high yield while reducing the corrosion of a reaction vessel, by reacting a copper (I) salt, a fluorooxo acid salt and an organic nitrogen compound with one another.

## Description

### TECHNICAL FIELD

The present invention relates to a method for producing a complex composed of an organic nitrogen compound and a copper(I) salt of a fluorooxo acid in high yield while allowing a reaction vessel to be hardly corroded.

### BACKGROUND ART

A complex composed of an organic nitrogen compound and a copper(I) salt of a fluorooxo acid such as tetrakis(acetonitrile)copper(I) tetrafluoroborate is useful in the production of electronic materials and the like, and there has been a need for a method of synthesizing the complex in high yield for the commercial production.

As a method for obtaining such a complex, for example, a method of using copper(I) oxide and allowing the copper(I) oxide to undergo a reaction at 70°C together with tetrafluoroboric acid and acetonitrile has hitherto been known (for example, see Patent Document 1). However, in this method, the yield based on the copper(I) oxide is as low as 74%, and there has been a demand for further heightening the yield in order to industrially produce the complex. Moreover, since a free strong acid such as tetrafluoroboric acid is used and allowed to undergo a reaction at 70°C in the method, a reaction vessel is easily corroded therewith and there has been a problem that such a glass-lined reaction vessel which is conventionally industrially widely used cannot be used.

Moreover, separately, a method of using copper(I) oxide and allowing the copper(I) oxide to undergo a reaction under reflux together with hexafluorophosphoric acid and acetonitrile (for example, see Non-Patent Document 1) has been known. However, in such a method, since hexafluorophosphoric acid, which is a free strong acid, is used and furthermore allowed to undergo a reaction under reflux, a reaction vessel is easily corroded therewith and there has also been a problem that the yield is low (for example, the yield based on the copper(I) oxide is 60%). Separately, although a technique of using copper(II) tetrafluoroborate in place of a free strong acid and allowing the copper(II) tetrafluoroborate to undergo a reaction under reflux together with copper and acetonitrile to produce a complex composed of an organic nitrogen compound and a copper(I) salt of a fluorooxo acid (for example, see Non-Patent Document 2) has also been known, a reaction vessel is easily corroded therewith since the reaction is performed under reflux, and it has still been difficult to efficiently obtain the complex since the yield is low.

Moreover, although the problem of the corrosion of a reaction vessel caused by a strong acid and a reaction at high temperatures can be eliminated by using a reaction vessel with high corrosion resistance such as Hastelloy-made equipment and the like, installing such facilities in the production line requires enormous production costs, and industrial-scale execution has not been easy.

From this kind of circumstance, the development of a production method which is capable of obtaining a complex composed of an organic nitrogen compound and a copper(I) salt of a fluorooxo acid in high yield, furthermore, allows a reaction vessel to be hardly corroded, and is high in practicality from the industrial standpoint has been eagerly desired.

### PRIOR ART DOCUMENTS

### PATENT DOCUMENT

Patent Document 1: DE Patent No. DE 1230025

### NON-PATENT DOCUMENTS

Non-Patent Document 1: Inorg. Synth., 19, 90 (1979)
Non-Patent Document 2: J. Org. Chem., 49, 608 (1984)

### SUMMARY OF THE INVENTION

### PROBLEMS TO BE SOLVED BY THE INVENTION

A main object of the present invention is to provide a method for producing a complex composed of an organic nitrogen compound and a copper(I) salt of a fluorooxo acid in high yield while allowing a reaction vessel to be hardly corroded.

### MEANS FOR SOLVING THE PROBLEM

As a result of extensive researches in view of solving the above-mentioned problem, the present inventors have found that, by allowing a copper(I) salt, a fluorooxo acid salt, and an organic nitrogen compound to react with one another, a complex composed of an organic nitrogen compound and a copper(I) salt of a fluorooxo acid can be obtained in high yield. Furthermore, the present inventors have found that, in the case of producing the complex by such a method, the corrosion of a reaction vessel is suppressed. The present invention has been completed by further researches on the basis of these findings.

That is, the present invention provides the following production method.
Item 1. A method for producing a complex composed of an organic nitrogen compound and a copper(I) salt of a fluorooxo acid, comprising the step of allowing a copper(I) salt represented by the following general formula (1)

   [Chemical 1] CuₐX (1)

   [wherein, X denotes a halogen atom, an oxo acid group, a cyano group, a thio acid group or a thiolate group, and a denotes a positive integer corresponding to the valence number of X],
   a fluorooxo acid salt represented by the following general formula (2)

   [Chemical 2] M_{b}Z_{c} (2)

   [wherein, M denotes an alkali metal, an alkaline earth metal or ammonium, Z denotes a fluorooxo acid group, and b and c denote required numbers for allowing the positive charge number and the negative charge number in the formula to coincide with each other], and
   an organic nitrogen compound represented by the following general formula (3)

   [Chemical 3] L (3)

   [wherein, L denotes a substituted or non-substituted aliphatic cyano compound with 1 to 6 carbon atoms, a substituted or non-substituted aromatic cyano compound, or a substituted or non-substituted nitrogen-containing heterocyclic compound] to react with one another.
Item 2. The method for producing a complex according to Item 1, wherein the copper(I) salt is copper(I) chloride and/or copper(I) bromide.
Item 3. The method for producing a complex according to Item 1 or 2, wherein the fluorooxo acid salt is at least one kind selected from the group consisting of sodium tetrafluoroborate, potassium tetrafluoroborate, sodium hexafluorosilicate, potassium hexafluorosilicate, sodium hexafluorophosphate and potassium hexafluorophosphate.
Item 4. The method for producing a complex according to any one of Items 1 to 3, wherein the organic nitrogen compound is acetonitrile.
Item 5. The method for producing a complex according to any one of Items 1 to 4, wherein the reaction is performed at -20 to 40°C.
Item 6. The method for producing a complex according to any one of Items 1 to 5, wherein the fluorooxo acid salt is used in a proportion of 0.5 to 10 molar equivalent relative to the copper(I) salt.
Item 7. The method for producing a complex according to any one of Items 1 to 6, wherein the organic nitrogen compound is used in a proportion of 1 to 50 molar equivalent relative to the copper(I) salt.
Item 8. The method for producing a complex according to any one of Items 1 to 7, wherein a reaction solvent used in the reaction is water, a polar solvent, or a mixed solvent thereof.
Item 9. The method for producing a complex according to any one of Items 1 to 5, wherein the complex composed of an organic nitrogen compound and a copper(I) salt of a fluorooxo acid is represented by the following general formula (4)

   [Chemical 4] [CuLₙ]_{d}Z (4)

   [wherein, Z and L represent the same meaning as above, n represents an integer of 2 to 6, and d denotes a positive integer corresponding to the valence number of Z].

### ADVANTAGES OF THE INVENTION

According to the production method of the present invention, it is possible to provide a method for producing a complex composed of an organic nitrogen compound and a copper(I) salt of a fluorooxo acid in high yield. Moreover, since a reaction vessel is hardly corroded in the production method of the present invention, and furthermore, the reaction is allowed to proceed at a temperature remarkably lower than that in the conventional method, it is unnecessary to use a reaction vessel which is high in corrosion resistance and heat resistance, and the production method of the present invention has a high usefulness even from the viewpoint of reduction of the investment costs for production facilities.

### EMBODIMENTS OF THE INVENTION

The present invention is a method for producing a complex composed of an organic nitrogen compound and a copper(I) salt of a fluorooxo acid, and is characterized as allowing a specific copper(I) salt, a specific fluorooxo acid salt, and a specific organic nitrogen compound to react with one another. Hereinafter, the production method of the present invention will be described in detail.

The copper(I) salt used in the production method of the present invention is represented by the following general formula (1).

[Chemical 5] CuₐX (1)

In the general formula (1), X denotes a halogen atom, an oxo acid group, a cyano group (CN), a thio acid group, or a thiolate group. Examples of the halogen atom include chlorine, bromine and the like. Examples of the oxo acid group include a sulfate group (SO₄), a nitrate group (NO₃), a borate group (BO₃), a phosphate group (PO₄), a carboxylate group (RCOO; for example, an acetate group (CH₃COO) and the like), and the like. Examples of the thio acid group include a thiocyano group (SCN), and the like. Examples of the thiolate group include a phenyl thiolate group (C₆H₅S), a trifluoromethyl thiolate group (CF₃S), and the like. Of these, from the viewpoints of the economical aspect and the yield, as the X, a halogen atom is preferred, and chlorine is further preferred.

In the general formula (1), a denotes a positive integer corresponding to the valence number of X.

The fluorooxo acid salt used in the production method of the present invention is represented by the following general formula (2).

[Chemical 6] M_{b}Z_{c} (2)

In the general formula (2), M denotes an alkali metal, an alkaline earth metal or ammonium. Examples of the alkali metal include sodium, potassium and the like. Examples of the alkaline earth metal include magnesium, calcium and the like. Of these, from the viewpoints of the economical aspect and the yield, as the M, an alkali metal is preferred, and sodium and potassium are further preferred.

In the general formula (2), Z denotes a fluorooxo acid group. Examples of the fluorooxo acid group include a tetrafluoroborate group (BF₄), a hexafluorosilicate group (SiF₆), a hexafluorophosphate group (PF₆), a hexafluorotitanate group (TiF₆), a hexafluorozirconate group (ZrF₆), a hexafluoroarsenate group (AsF₆), a hexafluorostannate group (SnF₆), and the like. Of these, a tetrafluoroborate group and a hexafluorophosphate group are preferred.

In the general formula (2), b and c denote required numbers for allowing the positive charge number and the negative charge number in the formula to coincide with each other.

Of these fluorooxo acid salts represented by the general formula (2), sodium tetrafluoroborate, potassium tetrafluoroborate, ammonium tetrafluoroborate, sodium hexafluorosilicate, potassium hexafluorosilicate, ammonium hexafluorosilicate, lithium hexafluorophosphate, sodium hexafluorophosphate, potassium hexafluorophosphate, and ammonium hexafluorophosphate are preferred, and sodium tetrafluoroborate, potassium tetrafluoroborate, sodium hexafluorosilicate, potassium hexafluorosilicate, sodium hexafluorophosphate, and potassium hexafluorophosphate are further preferred.

Although the proportion of the fluorooxo acid salt represented by the general formula (2) used is not particularly restricted, for example, the proportion is 0.5 to 10 molar equivalent, preferably 1 to 3 molar equivalent, relative to the copper(I) salt represented by the general formula (1). By allowing the proportion of the fluorooxo acid salt used to lie within the range, the degree of corrosion of a reaction vessel is further reduced. Moreover, when the proportion of the fluorooxo acid salt used is greater than or equal to 0.5 molar equivalent relative to the copper(I) salt, the reaction is allowed to efficiently proceed, and when the proportion is less than or equal to 10 molar equivalent, the proportion is preferred from the viewpoint of economy because an effect commensurate with the proportion thereof used is attained.

At the time of the reaction, the fluorooxo acid salt may be added directly, and may be dissolved in water or a polar solvent, or a mixed solvent thereof to be added. Examples of such a polar solvent include a kind of alcohol such as methanol, ethanol and isopropanol; a kind of glycol such as ethylene glycol and polyethylene glycol; and an aprotic polar solvent such as N,N'-dimethylformamide, dimethyl sulfoxide and N-methylpyrrolidone. Of these, from the viewpoint of economy, it is preferred that water be used alone.

The organic nitrogen compound used in the production method of the present invention is represented by the following general formula (3).

[Chemical 7] L (3)

In the general formula (3), L denotes a substituted or non-substituted aliphatic cyano compound with 1 to 6 carbon atoms, a substituted or non-substituted aromatic cyano compound, or a substituted or non-substituted nitrogen-containing heterocyclic compound.

Among the above-mentioned Ls, although the aliphatic cyano compound needs only to be an aliphatic compound having at least one cyano group and allow the carbon number thereof to lie within the range of 1 to 6, the carbon number is preferably 1 to 4, and further preferably 1 to 3. In this context, the carbon number of the aliphatic cyano compound refers to a carbon number of the aliphatic moiety in the aliphatic cyano compound in a state of being non-substituted (a carbon number excluding the carbon atom in the cyano group), and in the case of being substituted, the carbon number of the aliphatic cyano compound is a value determined without being added with the carbon number of the substituent.

Specifically, examples of the above-mentioned aliphatic cyano compound include acetonitrile, propiononitrile, isobutyronitrile, phenylacetonitrile, dichloroacetonitrile, succinonitrile, pentanenitrile, hexanedinitrile, cyclohexanecarbonitrile, acrylonitrile, allylnitrile, methacrylonitrile and the like. Of these aliphatic cyano compounds, acetonitrile, propionitrile and acrylonitrile are preferred.

Moreover, the above-mentioned aliphatic cyano compound may be a non-substituted one or may be a substituted one. In the case where the aliphatic cyano compound has a substituent, with regard to the kind, examples of the substituent include an alkyl group with 1 to 4, preferably 1 to 3, carbon atoms; an alkoxy group with 1 to 4, preferably 1 to 3, carbon atoms; a halogen atom, and the like. Moreover, in the case where the aliphatic cyano compound has a substituent, the number of the substituent is for example 1 to 4, preferably 1 to 3.

Among the above-mentioned Ls, the aromatic nitrogen compound needs only to be an aromatic compound having at least one nitrogen atom, and examples thereof include a compound formed by allowing a cyano group to be bonded to an aromatic compound, and preferably include a compound formed by allowing one or two cyano groups to be bonded to a benzene ring. Specifically, examples of such an aromatic nitrogen compound include benzonitrile, anisonitrile, methylbenzonitrile, chlorobenzonitrile, aminobenzonitrile, dicyanobenzene, and the like. Of these aromatic nitrogen compounds, benzonitrile and dicyanobenzene are preferred.

Moreover, the above-mentioned aromatic nitrogen compound may be a non-substituted one or may be a substituted one. In the case where the aromatic nitrogen compound has a substituent, with regard to the kind, examples of the substituent include an alkyl group with 1 to 4, preferably 1 to 3, carbon atoms; an alkoxy group with 1 to 4, preferably 1 to 3, carbon atoms; a halogen atom, and the like. Moreover, in the case where the aromatic nitrogen compound has a substituent, the number of the substituent is for example 1 to 4, preferably 1 to 3.

Among the above-mentioned Ls, the nitrogen-containing heterocyclic compound needs only to be a heterocyclic compound containing at least one nitrogen atom, and either of a monocyclic one and a combined cyclic one is acceptable. Specifically, examples of the nitrogen-containing heterocyclic compound include a compound having a nitrogen-containing heterocyclic skeleton such as a pyrrole skeleton, a pyrazole skeleton, an imidazole skeleton, a pyridine skeleton, an indole skeleton, a quinoline skeleton, an isoquinoline skeleton, a purine skeleton and a phenanthroline skeleton. Of these, a compound having a pyridine, pyrazole, imidazole or phenanthroline skeleton is preferred, and a compound having a pyridine, pyrazole or imidazole skeleton is further preferred. Further specifically, examples of such a nitrogen-containing heterocyclic compound include a compound having a pyridine skeleton such as pyridine, bipyridine, chloropyridine and cyanopyridine; a compound having an imidazole skeleton such as imidazole and methylimidazole; a compound having a pyrazole skeleton such as pyrazole and methylpyrazole; and a compound having a phenanthroline skeleton such as phenanthroline. Of these nitrogen-containing heterocyclic compounds, pyridine, bipyridine, cyanopyridine, imidazole, methylimidazole, pyrazole and methylpyrazole are preferred.

Moreover, the above-mentioned nitrogen-containing heterocyclic compound may be a non-substituted one or may be a substituted one. In the case where the nitrogen-containing heterocyclic compound has a substituent, with regard to the kind, examples of the substituent include an alkyl group with 1 to 4, preferably 1 to 3, carbon atoms; an alkoxy group with 1 to 4, preferably 1 to 3, carbon atoms; a halogen atom, and the like. Moreover, in the case where the nitrogen-containing heterocyclic compound has a substituent, the number of the substituent is for example 1 to 4, preferably 1 to 3.

Of the compounds represented by the general formula (3), acetonitrile, benzonitrile, dicyanobenzene, pyridine, bipyridine, cyanopyridine, imidazole, methylimidazole, pyrazole and methylpyrazole are preferred, and acetonitrile, benzonitrile and pyridine are further preferred.

Although the proportion of the organic nitrogen compound represented by the general formula (3) used is not particularly restricted, for example, the proportion is 1 to 50 molar equivalent, preferably 2 to 20 molar equivalent, relative to the copper(I) salt represented by the general formula (1). When the proportion of the organic nitrogen compound used is greater than or equal to 1 molar equivalent relative to the copper(I) salt, the reaction is allowed to efficiently proceed, and when the proportion is less than or equal to 50 molar equivalent, an effect commensurate with the proportion thereof used is attained.

As a reaction solvent used at the time of allowing the copper(I) salt represented by the general formula (1), the fluorooxo acid salt represented by the general formula (2), and the organic nitrogen compound represented by the general formula (3) to react with one another, water, a polar solvent or a mixed solvent thereof can be used, and specifically, examples thereof include one that is the same as the solvent used in the case of dissolving the fluorooxo acid salt to be added.

Although the amount of the above-mentioned reaction solvent used is not particularly restricted, for example, the amount is 200 to 5000 parts by mass, preferably 400 to 2000 parts by mass, relative to 100 parts by mass of the copper(I) salt represented by the general formula (1). When the proportion of the reaction solvent used is greater than or equal to 200 parts by mass relative to 100 parts by mass of the copper(I) salt represented by the general formula (1), there is no trouble in stirring nor possibility of causing lowering in yield, and moreover, when the proportion is less than or equal to 5000 parts by mass, there is also no considerable increase in amount of waste liquid.

In the production method of the present invention, compounds represented by the above-mentioned general formulas (1) to (3) are allowed to react with one another. At the time of the reaction, no particular limitation is put on the order of addition of the copper(I) salt, the fluorooxo acid salt and the organic nitrogen compound, the raw materials may be added in an arbitrary order.

Although the reaction temperature at the time of allowing compounds represented by the above-mentioned general formulas (1) to (3) to react with one another is not particularly restricted, from the viewpoint of suppressing the corrosion of equipment, the reaction temperature is preferably -20 to 40°C, further preferably 0 to 30°C.

With regard to the reaction time for the above-mentioned reaction, although the period of time varies depending on the reaction temperature, the reaction time is usually 0.1 to 30 hours or so, preferably 0.5 to 10 hours or so.

Moreover, the above-mentioned reaction may be performed under an inert atmosphere such as a nitrogen gas.

By allowing compounds represented by the above-mentioned general formulas (1) to (3) to react with one another in this way, a complex composed of an organic nitrogen compound and a copper(I) salt of a fluorooxo acid is formed. Specifically, examples of the above-mentioned complex obtained by the production method of the present invention include a complex shown in the following general formula (4).

[Chemical 8] [CuLₙ]_{d}Z (4)

In the general formula (4), Z and L represent the same meaning as above. Moreover, n represents the coordination number of L against the copper(I) ion, and although the number cannot be defined in a single uniform way since it varies depending on the reaction conditions employed, the number is usually 2 to 6, preferably 2 to 4. Moreover, in the general formula (4), d denotes a positive integer corresponding to the valence number of Z.

Specific examples of the complex shown in the foregoing general formula (4) obtained by the production method of the present invention include tetrakis(acetonitrile)copper(I) tetrafluoroborate, tetrakis(acetonitrile)copper(I) hexafluorophosphate, tetrakis(propiononitrile)copper(I) tetrafluoroborate, tetrakis(benzonitrile)copper(I) tetrafluoroborate, tetrakis(anisonitrile)copper(I) tetrafluoroborate, bis(acrylonitrile)copper(I) tetrafluoroborate, bis(cyanopyridine)copper(I) tetrafluoroborate, bis(2,2'-bipyridine)copper tetrafluoroborate, and the like. Of these complexes, in particular, the production method of the present invention is suitable for the production of tetrakis(acetonitrile)copper(I) tetrafluoroborate, tetrakis(benzonitrile)copper(I) hexafluorophosphate, tetrakis(pyridine)copper(I) tetrafluoroborate, or bis(2-cyanopyridine)copper(I) tetrafluoroborate.

After the completion of the above-mentioned reaction, the resultant complex composed of an organic nitrogen compound and a copper(I) salt of a fluorooxo acid needs only to be isolated according to a conventionally known method. For example, after the completion of the above-mentioned reaction, the precipitated crystals are filtered off and washed with a solvent used in the reaction, after which, by allowing the crystals to be dried, the complex can be recovered in high purity.

The complex composed of an organic nitrogen compound and a copper(I) salt of a fluorooxo acid which is produced by the production method of the present invention can be used in a technical field (for example, the production of electronic materials, and the like) where these complexes have hitherto been used.

### EXAMPLES

The present invention will be described below in more detail with reference to examples, but the present invention is not limited only to these examples.

### [Example 1]

### Synthesis of tetrakis(acetonitrile)copper(I) tetrafluoroborate

In a 2000-mL four-necked flask equipped with a stirrer, a thermometer and a condenser, 50 g (0.5 mol) of copper(I) chloride, 103 g (2.5 mol) of acetonitrile and 500 g of water were placed, and 366 g (1.0 mol) of an aqueous 30% by mass sodium tetrafluoroborate solution was added to the contents at 20 to 30°C, after which the contents were allowed to react for 1 hour at the same temperature. The precipitated crystals were filtered, after which the crystals were washed with 50% by mass ethanol-water and dried to obtain 149 g of tetrakis(acetonitrile)copper(I) tetrafluoroborate.

The yield of the obtained tetrakis(acetonitrile)copper(I) tetrafluoroborate based on the copper(I) chloride was determined to be 95%. Moreover, the elemental analysis value of the obtained tetrakis(acetonitrile)copper(I) tetrafluoroborate was determined to be Cu; 20.4%, B; 3.4%, N; 17.8%, and corresponded to the theoretical value (Cu; 20.2%, B; 3.4%, N; 17.8%).

### [Example 2]

### Synthesis of tetrakis(benzonitrile)copper(I) hexafluorophosphate

In a 2000-mL four-necked flask equipped with a stirrer, a thermometer and a condenser, 50 g (0.5 mol) of copper(I) chloride, 258 g (2.5 mol) of benzonitrile and 500 g of water were placed, and 920 g (1 mol) of an aqueous 20% by mass potassium hexafluorophosphate solution was added to the contents at 20 to 30°C, after which the contents were allowed to react for 5 hours at the same temperature. The precipitated crystals were filtered, after which the crystals were washed with 50% by mass ethanol-water and dried to obtain 267 g of tetrakis(benzonitrile)copper(I) hexafluorophosphate.

The yield of the obtained tetrakis(benzonitrile)copper(I) hexafluorophosphate based on the copper(I) chloride was determined to be 86%. Moreover, the elemental analysis value of the obtained tetrakis(benzonitrile)copper(I) hexafluorophosphate was determined to be Cu; 10.1%, B; 5.0%, N; 9.0%, and corresponded to the theoretical value (Cu; 10.2%, P; 5.0%, N; 9.0%).

### [Example 3]

### Synthesis of bis(2-cyanopyridine)copper(I) tetrafluoroborate

In a 2000-mL four-necked flask equipped with a stirrer, a thermometer and a condenser, 50 g (0.5 mol) of copper(I) chloride, 156 g (1.5 mol) of 2-cyanopyridine and 500 g of water were placed, and 366 g (1.0 mol) of an aqueous 30% by mass sodium tetrafluoroborate solution was added to the contents at 20 to 30°C, after which the contents were allowed to react for 3 hours at the same temperature. The precipitated crystals were filtered, after which the crystals were washed with 50% by mass ethanol-water and dried to obtain 156 g of bis(2-cyanopyridine)copper(I) tetrafluoroborate.

The yield of the obtained bis(2-cyanopyridine)copper(I) tetrafluoroborate based on the copper(I) chloride was determined to be 87%. Moreover, the elemental analysis value of the obtained bis(2-cyanopyridine)copper(I) tetrafluoroborate was determined to be Cu; 17.7%, B; 3.0%, N; 15.6%, and corresponded to the theoretical value (Cu; 17.7%, B; 3.0%, N; 15.6%).

### [Example 4]

According to Example 1, in a 500-mL Teflon-coated separable flask equipped with a stirrer, a thermometer, a condenser and a glass-lined test piece disposed so as to be immersed in a reaction liquid, 10 g (0.1 mol) of copper(I) chloride, 21 g (0.5 mol) of acetonitrile and 100 g of water were placed, and 73 g (0.2 mol) of an aqueous 30% by mass sodium tetrafluoroborate solution was added to the contents at 20 to 30°C, after which the contents were allowed to react for 100 hours at the same temperature. The corrosion rate was calculated from the surface area of the test piece, the specific gravity of glass lining, and the weight change in the test piece before and after the reaction, whereupon the corrosion rate of glass lining was determined to be less than 0.1 mm/year, and it was confirmed that there is no problem in the use of a glass-lined reaction pot.

### [Comparative Example 1]

According to the conditions described in Patent Document 1, in a 2000-mL four-necked flask equipped with a stirrer, a thermometer and a condenser, 36 g (0.25 mol) of copper(I) oxide, 625 ml of water and 115 ml of acetonitrile were placed, and 125 ml (1.0 mol) of an aqueous 31.4% tetrafluoroboric acid solution was added to the contents at 70°C, after which insoluble materials were removed by filtration, and furthermore, the contents were allowed to react for 5 hours at the same temperature. The precipitated crystals were filtered, after which the crystals were washed with ethanol and ethyl ether and dried to obtain 60 g of tetrakis(acetonitrile)copper(I) tetrafluoroborate. The yield of the obtained tetrakis(acetonitrile)copper(I) tetrafluoroborate based on the copper(I) chloride was determined to be 76%.

### [Comparative Example 2]

According to the conditions described in Patent Document 1, in a 500-mL Teflon-coated separable flask equipped with a stirrer, a thermometer, a condenser and a glass-lined test piece disposed so as to be immersed in a reaction liquid, 14 g (0.1 mol) of copper(I) oxide, 250 ml of water and 46 ml of acetonitrile were placed, and 50 ml (0.4 mol) of an aqueous 31.4% tetrafluoroboric acid solution was added to the contents at 70°C, after which the contents were allowed to react for 100 hours at the same temperature. The corrosion rate was calculated from the surface area of the test piece, the specific gravity of glass lining, and the weight change in the test piece before and after the reaction, whereupon the corrosion rate of glass lining was determined to be greater than or equal to 5 mm/year, and it was confirmed that using a glass-lined reaction pot in the production method of Patent Document 1 is difficult.

### [Comparative Example 3]

According to the conditions described in Non-Patent Document 2, in a 500-mL Teflon-coated separable flask equipped with a stirrer, a thermometer, a condenser and a glass-lined test piece disposed so as to be immersed in a reaction liquid, 24 g (0.1 mol) of copper(II) tetrafluoroborate, 200 ml of acetonitrile, and 7 g (0.1 mol) of copper powder were placed, after which the contents were allowed to react for 100 hours under reflux. The corrosion rate was calculated from the surface area of the test piece, the specific gravity of glass lining, and the weight change in the test piece before and after the reaction, whereupon the corrosion rate of glass lining was determined to be greater than or equal to 5 mm/year, and it was confirmed that using a glass-lined reaction pot in the production method of Non-Patent Document 2 is difficult.

## Claims

1. A method for producing a complex composed of an organic nitrogen compound and a copper(I) salt of a fluorooxo acid, comprising the step of allowing a copper(I) salt represented by the following general formula (1)
[Chemical 1] CuₐX (1)
[wherein, X denotes a halogen atom, an oxo acid group, a cyano group, a thio acid group or a thiolate group, and a denotes a positive integer corresponding to the valence number of X],
a fluorooxo acid salt represented by the following general formula (2)
[Chemical 2] M_{b}Z_{c} (2)
[wherein, M denotes an alkali metal, an alkaline earth metal or ammonium, Z denotes a fluorooxo acid group, and b and c denote required numbers for allowing the positive charge number and the negative charge number in the formula to coincide with each other], and
an organic nitrogen compound represented by the following general formula (3)
[Chemical 3] L (3)
[wherein, L denotes a substituted or non-substituted aliphatic cyano compound with 1 to 6 carbon atoms, a substituted or non-substituted aromatic cyano compound, or a substituted or non-substituted nitrogen-containing heterocyclic compound] to react with one another.

2. The method for producing a complex according to claim 1, wherein the copper(I) salt is copper(I) chloride and/or copper(I) bromide.

3. The method for producing a complex according to claim 1 or 2, wherein the fluorooxo acid salt is at least one kind selected from the group consisting of sodium tetrafluoroborate, potassium tetrafluoroborate, sodium hexafluorosilicate, potassium hexafluorosilicate, sodium hexafluorophosphate and potassium hexafluorophosphate.

4. The method for producing a complex according to any one of claims 1 to 3, wherein the organic nitrogen compound is acetonitrile.

5. The method for producing a complex according to any one of claims 1 to 4, wherein the reaction is performed at -20 to 40°C.

6. The method for producing a complex according to any one of claims 1 to 5, wherein the fluorooxo acid salt is used in a proportion of 0.5 to 10 molar equivalent relative to the copper(I) salt.

7. The method for producing a complex according to any one of claims 1 to 6, wherein the organic nitrogen compound is used in a proportion of 1 to 50 molar equivalent relative to the copper(I) salt.

8. The method for producing a complex according to any one of claims 1 to 7, wherein a reaction solvent used in the reaction is water, a polar solvent, or a mixed solvent thereof.

9. The method for producing a complex according to any one of claims 1 to 5, wherein the complex composed of an organic nitrogen compound and a copper(I) salt of a fluorooxo acid is represented by the following general formula (4)
[Chemical 4] [CuLₙ]_{d}Z (4)
[wherein, Z and L represent the same meaning as above, n represents an integer of 2 to 6, and d denotes a positive integer corresponding to the valence number of Z].
